(19) Europäisches Patentamt / European Patent Office / Office européen des brevets

(11) **EP 4 317 405 A1**

(12) **EUROPEAN PATENT APPLICATION**
published in accordance with Art. 153(4) EPC

(43) Date of publication:
**07.02.2024 Bulletin 2024/06**

(21) Application number: **22876683.8**

(22) Date of filing: **26.08.2022**

(51) International Patent Classification (IPC):
**C12M 1/34** $^{(2006.01)}$    **G01N 21/25** $^{(2006.01)}$
**G16C 20/30** $^{(2019.01)}$    **G16C 20/70** $^{(2019.01)}$

(52) Cooperative Patent Classification (CPC):
**C12M 1/34; G01N 21/25; G16C 20/30; G16C 20/70**

(86) International application number:
**PCT/KR2022/012789**

(87) International publication number:
**WO 2023/054908 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2021 KR 20210128110**

(71) Applicant: **LG Chem, Ltd.**
**Seoul 07336 (KR)**

(72) Inventors:
• **CHOI, Jae Gil**
**Daejeon 34122 (KR)**

• **KWON, You Bin**
**Daejeon 34122 (KR)**
• **SONG, Young Soo**
**Daejeon 34122 (KR)**
• **IM, Ye Hoon**
**Daejeon 34122 (KR)**
• **CHOI, Jun Won**
**Daejeon 34122 (KR)**

(74) Representative: **Goddar, Heinz J. et al**
**Boehmert & Boehmert**
**Anwaltspartnerschaft mbB**
**Pettenkoferstrasse 22**
**80336 München (DE)**

(54) **METHOD AND SYSTEM FOR PREDICTING MICROBIAL CULTURE CONCENTRATION**

(57) The present invention relates to a microorganism culture production amount prediction system and method with improved accuracy in predicting the microorganism culture production amount using a metabolic model equation by calculating the predicted substrate concentration from the metabolic model equation, calculating the actually measure substrate concentration from the spectrum data of the culture solution, comparing the predicted substrate concentration with the actually measured substrate concentration, and updating the parameters of the metabolic model equation, in a method for predicting the culture concentration of microorganisms with known metabolic model equation governing the metabolic model.

**FIG. 1**

## Description

### TECHNICAL FIELD

The present invention relates to a method and system for predicting a microorganism culture concentration in real time during a microorganism culture process.

### BACKGROUND ART

Recently, in the global trend that emphasizes eco-friendly products, the production of bioplastics made from renewable raw materials rather than fossil fuel-based monomers such as petroleum and natural gas is increasing.

Bioplastics have an advantage of being free from waste problems as the bioplastics emit less carbon dioxide in a production process than petroleum-based plastics and decompose naturally within a few years depending on materials and products.

For the production of bioplastics, after adjusting a growth environment of microorganism and increasing the number of microorganisms through culturing, a step of producing bioplastic raw materials through cell metabolism is performed thoroughly. In this process, it is necessary to adjust an optimal growth environment for the growth and metabolism of the microorganism.

It is necessary to consider various conditions, such as concentration of substrate used as food for the microorganism, concentration of oxygen, feed injection method, temperature, pH, depending on the type of microorganism, and conventionally, these conditions were mainly found through experiments. However, inefficiencies in time and cost were a problem in adjusting these process variables through actual experiments.

Under this background, in order to measure a concentration of the microorganism during the culture process, there was a method of estimating a parameter of a metabolic model equation from a culture experiment using a metabolic model equation, setting an arbitrary initial value for the parameter, and predicting a microorganism concentration during the process from the metabolic model equation.

However, in the case of this prior art, there was a problem in that accurate estimation was not possible due to a change in conditions during the process, and a method for improving this problem was needed.

The related prior art is described in the following patent documents.

Korean patent application No. 10-2020-7013036
Korean patent application No. 10-2020-7004943

### DISCLOSURE OF THE INVENTION

### TECHNICAL PROBLEM

The present invention is to solve the problems described above, and to provide a method and system for predicting concentration information of the microorganism in real time during a microorganism culture process.

### TECHNICAL **SOLUTION**

In order to solve the problems described above, the present invention provides a microorganism culture production amount prediction system comprising an incubator for culturing a microorganism in a culture solution, a spectrum acquisition unit that acquires a real-time spectrum of the culture solution, a memory device that stores a concentration actual-measurement model, which is an artificial intelligence model trained to calculate an actually measured substrate concentration from spectrum data, and a metabolic model equation prescribing a metabolic activity of the microorganism, and a control unit that calculates a predicted value of microorganism production amount from the concentration actual-measurement model and the metabolic model equation, in which the control unit calculates the actually measured substrate concentration by receiving the spectrum data from the spectrum acquisition unit and inputting the spectrum data to the concentration actual-measurement model, updates parameters of the metabolic model equation from the actually measured substrate concentration and predicted substrate concentration data by calculating a predicted substrate concentration from the metabolic model equation, and calculates the predicted value of the microorganism production amount from the metabolic model equation with the updated parameters.

In addition, the present invention provides a microorganism culture concentration prediction device comprising a spectral measurement unit that acquires spectrum data of a culture solution of a microorganism incubator, a microorganism production amount prediction unit that calculates an actually measured substrate concentration in a culture solution by inputting the spectrum data into a concentration actual-measurement model and predicts a microorganism culture concentration by updating a parameter of a microorganism metabolism model using the actually measured substrate concentration. In this time, the microorganism production amount prediction unit is configured to include a substrate concentration actual-measurement unit that calculates an actually measured substrate concentration from the spectrum data, a metabolic model unit that calculates a predicted substrate concentration from a metabolic model equation prescribing a metabolic activity of the microorganism, and a parameter update unit that updates parameters of the metabolic model equation from the actually measured substrate concentration and the predicted substrate concentration, and the metabolic model unit may include a microorganism production amount prediction unit that applies the parameters updated by the parameter update unit to the metabolic model equation, and calculates a microorganism culture concentration predicted value from the metabolic model equation to which the updated parameters are applied.

In addition, the substrate concentration actual-measurement unit includes a concentration actual-measurement model machine-learned to calculate a concentration of the substrate from the spectrum data, and inputs the real-time spectrum data into the concentration actual-measurement model to calculate the actually measured substrate concentration. In this case, the metabolic model equation includes a concentration calculation model equation for predicting a concentration of Escherichia coli cells in the culture solution, and a substrate concentration calculation model equation for calculating a concentration of glucose as a substrate.

In addition, the present invention provides a microorganism culture concentration prediction method in a culture process, the method comprising a spectrum data acquisition step of acquiring a real-time spectrum of a culture solution of a microorganism incubator, an actually measured substrate concentration calculation step of calculating an actually measured substrate concentration in a culture solution by inputting the real-time spectrum data into a pre-trained concentration actual-measurement model, a predicted substrate concentration calculation step of calculating a predicted substrate concentration of a substrate from a metabolic model equation of the microorganism, a parameter update step of updating the parameters of the metabolic model equation so that the predicted substrate concentration matches the actually measured substrate concentration by comparing the actually measured substrate concentration calculated by the actually measured substrate concentration calculation unit with the predicted substrate concentration calculated through the metabolic model unit, and a microorganism production amount prediction step of predicting a microorganism production amount from the metabolic model equation to which the updated parameters are applied.

In this case, in the predicted substrate concentration calculation step, the predicted substrate concentration from the metabolic model equation is calculated by estimating the parameters of the metabolic model equation from concentration data of each component in the culture solution obtained in advance, and applying the estimated parameter, and the metabolic model equation includes a concentration calculation model equation for predicting the concentration of Escherichia coli cells in the culture solution, and a substrate concentration calculation model equation for calculating a concentration of glucose as a substrate.

## ADVANTAGEOUS EFFECTS

The present invention has an effect of improving the accuracy of calculating a microorganism culture concentration by updating the parameters of the metabolic model equation using real-time substrate concentration in calculating the microorganism culture concentration according to the metabolic model equation.

## BRIEF DESCRIPTION OF THE DRAWINGS

FIG. 1 is a block diagram of a system for predicting a microorganism concentration according to the present invention.
FIG. 2 is a diagram for describing each procedure of microorganism concentration prediction according to the present invention.
FIG. 3 is a metabolic model illustrating a metabolic relationship between culture and production of Escherichia coli.
FIG. 4 is a graph illustrating a predicted substrate concentration value and an actually measured substrate concentration value in an example of the present invention.
FIG. 5 is a graph illustrating predicted concentration values of respective components according to a metabolic model equation with an updated parameter according to the present invention.

## MODE FOR CARRYING OUT THE INVENTION

Hereinafter, embodiments of the present invention will be described in detail with reference to the accompanying drawings so that those of ordinary skill in the art can easily embody the present invention. However, the present invention may be implemented in various different forms and is not limited to the embodiments described herein. In order to clearly describe the present invention in the drawings, parts irrelevant to the description are omitted, and similar reference numerals are attached to similar parts throughout the specification.

In the present invention, learning or training means training a neural network in which a computer algorithm that allows a predetermined output value is output with respect to an input value in the field of artificial intelligence or such an algorithm is implemented in the form of a neural network, and refers to one of the processes of performing a neural network algorithm by a computer computing device. In addition, in the present invention, the term 'creation of a model' means to train a neural network or a computer algorithm implementing the neural network before being trained, and generate a computer algorithm that has been trained to generate a desired output value with respect to an input value.

The present invention relates to a method and system for predicting a microorganism culture concentration in an incubator.

More specifically, the present invention relates to the prediction of the culture concentration of the microorganism whose metabolic pathway have been identified through genetic manipulation, and includes a spectrometer capable of supplying a substrate used as food for the microorganism to the incubator and obtaining a real-time spectrum of the substrate in a culture solution. In addition, the present invention is configured to include a pre-trained machine learning algorithm capable of calculating the actually measured concentration of the substrate by learning the real-time spectrum data of the spectrometer, and includes a computer algorithm that predicts the concentration of a substrate and the microorganism concentration through a metabolic model equation based on the identified metabolic pathway. The present invention includes a method and system for updating the parameters of the metabolic model equation using an optimization algorithm by comparing the calculated actually measured concentration of the substrate with the substrate concentration predicted value through the metabolic model equation, and predicting the concentration of microorganism by applying the updated parameters to the metabolic model equation.

According to the present invention, in the case of predicting the microorganism concentration based on the metabolic model equation, matters that a parameter setting value is fixed to an initial setting value are improved, the substrate concentration is measured in real time through the spectrum data, and the parameters of the metabolic model equation are updated based on this, thereby providing a more accurate metabolic model equation-based concentration prediction technique.

Hereinafter, the present invention will be described in detail with reference to the drawings.

1. Microorganism culture concentration prediction system according to the present invention

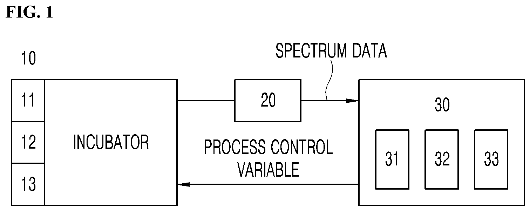

The microorganism culture concentration prediction system according to the present invention will be described with reference to FIG. 1.

The microorganism culture concentration prediction system of the present invention is configured to include a microorganism incubator 10, and a spectrometer 20 that acquires real-time spectrum data of the culture solution of the microorganism incubator, a substrate concentration actual-measurement unit 31 that inputs the real-time spectrum data to a pre-trained concentration prediction model, and calculates an actually measured substrate concentration in the culture solution, a metabolic model unit 32 which is mounted with a metabolic model of the microorganism and calculates a concentration of the substrate according to the metabolic model, and a parameter update unit 33 that compares the actually measured substrate concentration calculated from the actually measured substrate concentration calculation unit with a predicted substrate concentration calculated through the metabolic model unit to calculate and update parameters of the metabolic model of the metabolic model unit so that the predicted substrate concentration matches the actually measured substrate concentration, and is configured to include a microorganism production amount prediction unit 30 that predicts a microorganism production amount from the metabolic model with the updated parameters by applying the updated parameters.

The microorganism in the example of the present invention may mean Escherichia coli.

1.1. Culture unit 10

The microorganism culture unit of the present invention is configured to include, in addition to each configuration of the normal incubator, a dissolved oxygen sensor 11 that measures a dissolved oxygen concentration in a culture chamber in which culture progresses, a feeding amount measurement unit 12 that measures a glucose feeding amount, a volume measurement unit 13 that measures the volume of the culture solution.

(1) Dissolved oxygen sensor 11

The dissolved oxygen sensor measures the dissolved oxygen concentration in the culture chamber and transmits the dissolved oxygen concentration to the control unit.

(2) Feeding amount measurement unit 12

It transmits the glucose feeding amount to be supplied to the culture chamber to the control unit.

(3) Volume measuring unit 13

It measures the volume of the culture solution in the culture chamber and transmit the volume of the culture solution to the control unit.

1.2. Spectral measurement unit 20

It is a configuration for acquiring the spectrum data of the culture solution in the incubator, and acquires the spectrum data of the culture solution using a known spectrometer. 1.3. Microorganism production amount prediction unit 30

The microorganism production amount prediction unit calculates the parameters of the metabolic model equation of the microorganism metabolism model from the actually measured substrate concentration calculated from the spectrum data and predicts the microorganism concentration from the microorganism metabolism model equation by applying the calculated parameters to predict the microorganism production amount. The microorganism production amount prediction unit outputs a production amount as the predicted microorganism concentration, and adjusts and outputs an incubator process control variable using the production amount.

The microorganism production amount prediction unit 30 may be physically configured with a memory device that stores a concentration actual-measurement model, a metabolic model equation, and optimization algorithm, and a control unit processor that performs operations such as calculation of actually measured substrate concentration, calculation of microorganism concentration, calculation/update of parameters, etc. (1) Substrate concentration actual-measurement unit 31.

It is a configuration for measuring the actually measured substrate concentration from the spectrum data of the culture solution. The substrate concentration actual-measurement unit is mounted with a machine-learned concentration actual-measurement model to calculate the concentration of the substrate from the spectrum data of the culture solution, and calculates the actually measured substrate concentration from the concentration of the substrate.

The substrate concentration actual-measurement unit is mounted with a known predictive model such as a partial least squares (PLS) model that calculates the concentration of substrate from the spectrum data of the culture solution as a concentration actual-measurement model to receive the spectrum data and calculates the concentration of the substrate therefrom. In the present invention, a concentration value of the substrate calculated through the concentration actual-measurement model from the spectrum data is defined as the actually measured substrate concentration.

(2) Metabolic model unit 32

The microorganism production amount prediction unit 30 includes a metabolic model unit 32. The metabolic model unit is mounted with a computer-executable algorithm for calculating the concentration of the substrate according to a metabolic model equation that prescribes a metabolic activity of the microorganism culture as the metabolic model equation. A substrate concentration calculation model equation as the metabolic model equation mounted in the metabolic model unit includes predetermined parameters and includes a substance balance equation expressed as a differential equation, and the concentration of each component of the culture solution calculated by the metabolic model unit is defined as a predicted concentration in the present invention.

The metabolic model unit outputs two output values.

Firstly, the parameters of the metabolic model equation are estimated from the concentration values of each component obtained through a culture experiment, and the predicted substrate concentration value calculated by applying the estimated parameters to the substrate concentration model equation is output. The predicted substrate concentration value is compared with the actually measured substrate concentration and applied to the parameter update by the parameter update unit to be described later.

Secondly, the updated parameter from the parameter update unit to be described later is received and applied to a microorganism cell concentration model equation of the metabolic model equation, and a microorganism concentration predicted value by the microorganism cell concentration model equation having the updated parameters is calculated and output as the microorganism production amount.

(3) Parameter update unit 33

The parameter update unit updates respective parameters of the metabolic model equation using the calculated actually measured substrate concentration and predicted substrate concentration, and transmits the updated parameters to the metabolic model unit. The update of the parameters of the metabolic model equation is updated so that the actually measured substrate concentration value and the predicted substrate concentration value match as much as possible, and a specific method thereof will be described in the microorganism culture concentration prediction method of the present invention.

2. Microorganism culture concentration prediction method in the culture solution according to the present invention

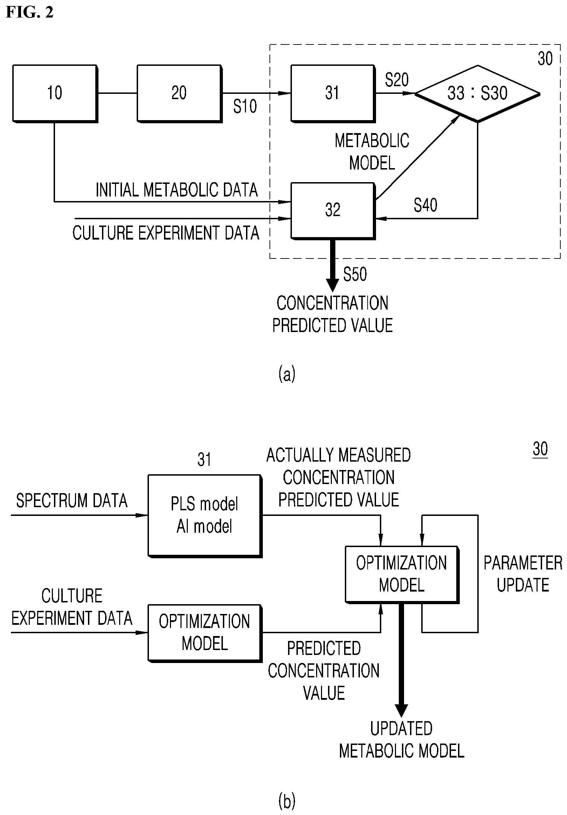

The microorganism culture concentration prediction method in the culture solution according to the present invention consists of procedures performed by respective configuration units of the microorganism culture concentration prediction system described above. Each procedure will be described with reference to FIG. 2.

(1) Real-time spectrum data acquisition step (S10)

It is a step of acquiring real-time spectrum data from the culture solution of the culture unit from the spectral measurement unit 20.

(2) Actually measured substrate concentration calculation step (S20)

It is a step of actually measuring the concentration of the substrate by inputting the obtained real-time spectrum data into the substrate concentration actual-measurement unit. The real-time spectrum data is input to the pre-trained concentration actual-measurement model of the substrate concentration actual-measurement unit 31, and the actually measured substrate concentration value is output.

(3) Predicted substrate concentration calculation step (S30)

The predicted substrate concentration is calculated according to the substrate concentration metabolic model equation mounted in the metabolic model unit. For the predicted substrate concentration, the concentration data of each component in the culture solution, which is obtained in advance through a culture experiment to be described later is substituted into a metabolic model equation composed of a differential equation and a rate equation, and the parameters of the metabolic model equation are estimated through a known optimization technique are estimated.
As optimization algorithms, a least squares method & weighted least squares method, Gradient Descent search method, Newton method (Newton-Raphson method), Gauss-Newton method, Levenberg-Marquardt method, genetic algorithm, ant colony optimization, quenching algorithm, mimetic algorithm, evolutionary computation method, evolutionary strategy method, evolutionary programming are known, and the present invention is not limited to being applied to a specific algorithm.
Then, the predicted substrate concentration is calculated from the substrate concentration metabolic model equation by substituting the estimated parameters into the metabolic model equation.

(4) Parameter update step (S40)

It is a procedure for updating the parameter values of the metabolic model equations of the metabolic model unit 32 using the actually measured substrate concentration value and the calculated predicted substrate concentration value.
The parameter update is a step of updating respective parameter values of the metabolic model equation estimated in the predicted substrate concentration calculation step so that the predicted substrate concentration according to the metabolic model and the actually measured substrate concentration are optimally matched by comparing the time-series measured substrate concentration value with the predicted substrate concentration value and applying the known optimization algorithm such as the genetic algorithm.

(5) Microorganism production amount prediction step (S50)

It is a process of outputting the predicted value of the microorganism production amount by calculating the microorganism concentration from the metabolic model equation of microorganism cell concentration with the updated parameter value.

<Example>

An example according to the present invention will be described.

(1) Acquisition of spectrum data and calculation of actually measured concentration

First, the actually measured concentration of the substrate is calculated in time-series by inputting the spectrum data obtained in time-series into the actually measured substrate concentration calculation model obtained by being subjected to machine learning by using the concentration value of the substrate in the culture medium, which is obtained through a known PLS model or culture experiment from the spectrum data obtained from the incubator, and the spectrum data as training data,

The above culture experiment was carried out as an experiment with the following culture experiment conditions.

<Culture experiment conditions for parameter estimation and actually measured substrate concentration calculation model training>

    Strain: E. coli W3110
    Initial dry cell weight (DCW): 0.1887 g/L
    Initial substrate concentration: 20 g/L
    Culture temperature 35°C, the number of revolutions of incubator: 500 ~ 900 rpm
    Culture solution pH: 6.95 (22.2% NH4OH (ammonia water: pH adjustment solution))
    Feeding Rate: 50ml/h Continuous Feeding after 10hr (No feeding before 10hr)
    Feeding Solution: Glucose 700 g/L, MgSO$_4$ (magnesium sulfate) 15g/L, Trace metal solution 10ml/L

(2) Metabolic model equation parameter calculation and predicted substrate concentration calculation

<Metabolic model equation applied to the present invention>

In the example of the present invention, the Escherichia coli metabolic model equation is mounted on the metabolic model unit, the metabolic model equation includes a concentration calculation model equation (Equation 1) of Escherichia coli cells in the culture solution, a substrate concentration calculation model equation (Equation 2) for calculating the concentration of glucose as a substrate, a produced-acetate concentration calculation model equation (Equation 3), and a dissolved oxygen concentration calculation model equation (Equation 4) for calculating a dissolved oxygen amount, and consists of a balance equation and rate equation for calculating the concentration of Escherichia coli, glucose, and acetate, and the amount of dissolved oxygen.

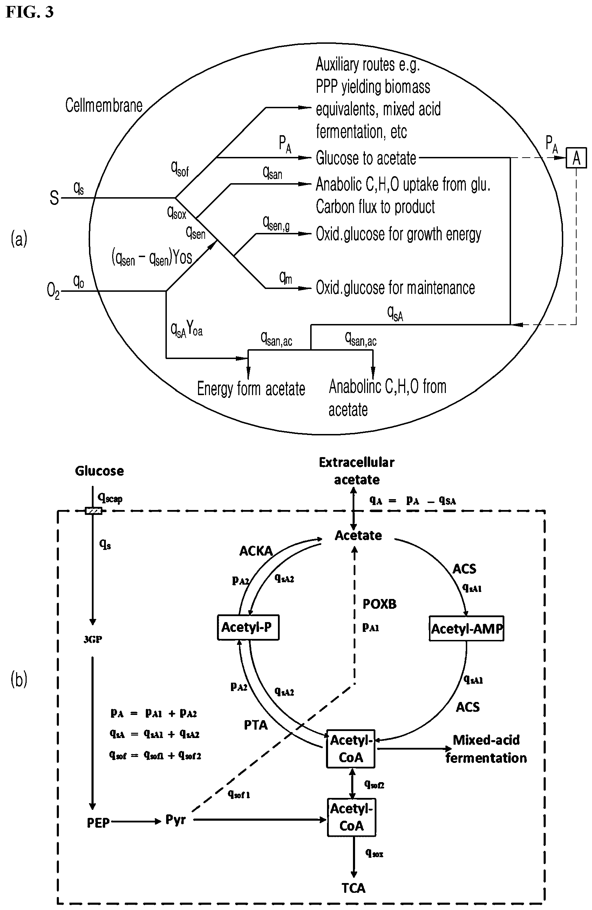

The Escherichia coli metabolic model equation applied in the example of the present invention is based on a macrodynamic model (FIG. 3(a)) and the acetate circulation system model (FIG. 3(b)) related to the Escherichia coli culture proposed in Modelling overflow metabolism in Escherichia coli by acetate cycling, Biochemical Engineering Journal 125 (2017) 23-30, Emmanuel Anane, et, al.

Equation 1

$$\frac{dX}{dt} = \frac{F}{V}(0 - X) + \mu X \quad , \quad \mu = (q_{sox} - q_m)Y_{em} + q_{sof}Y_{xsof} + q_{sA}Y_{xa}$$

Equation 2

$$\frac{dS}{dt} = \frac{F}{V}(S_i - S) - q_sX \quad , \quad q_s = \frac{q_{smax}}{1 + \frac{A}{K_{ia}}} \cdot \frac{S}{S + K_s} \quad , \quad q_{sox} = (q_s - q_{sof}) \cdot \frac{DOT}{DOT + K_o} \quad , \quad q_{sof} = \frac{P_{Amax}q_s}{q_s + K_{ap}}$$

Equation 3

$$\frac{dA}{dt} = \frac{F}{V}(0 - A) + q_A X \qquad q_A = p_A - q_{sA}, \qquad p_A = q_{sof} Y_{as}, \qquad q_{sA} = \frac{q_{Amax}}{1 + \frac{q_s}{K_{is}}} \cdot \frac{A}{A + K_{sa}}$$

Equation 4

$$\frac{dDO_a}{dt} = K_{la}(DO^* - DO_a) - q_o XH \qquad q_o = (q_{sox} - q_m)Y_{os} + q_{sA}Y_{oa},$$

In the above equations, X represents the concentration of Escherichia coli cells, S represents the concentration of glucose as a substrate, A represents the acetate concentration, F represents the feed, V represents the volume, $\mu$ represents the growth rate constant, $q_{sox}$ represents the rate of glucose uptake through oxidative metabolism, $q_{sof}$ represents the rate of glucose uptake through excess metabolism, $q_{sA}$ represents the rate of glucose uptake through acetate metabolism, $q_m$ represents the cell maintenance constant, $Y_{em}$ represents the yield excluding cell maintenance, $Y_{xsof}$ represents production yield through the excess metabolic pathway of cells/glucose, $Y_{xa}$ represents acetate/cell production yield, $q_{smax}$ represents the maximum glucose uptake rate constant, $K_{ia}$ and $K_s$ represent the acetate-induced glucose uptake inhibitory constant, the glucose-induced acetate uptake inhibitory constant, respectively, $q_{sox}$ represents the rate of glucose uptake through oxidative metabolism, $q_{sof}$ represents the rate of glucose uptake through excess metabolism, $P_{Amax}$ represents the maximum acetate production rate constant, Ko represents the oxygen consumption affinity constant, $K_{ap}$ represents the intracellular acetate production saturation constant (monod type), $q_A$ represents the rate of acetate consumption, $p_A$ represents the rate of acetate production, $q_{sA}$ represents the rate of glucose uptake through acetate metabolism, $q_{sof}$ represents the rate of glucose uptake through excess metabolism, $Y_{as}$ represents the acetate production yield through excess metabolism (acetate/glucose), $q_{Amax}$ represents the maximum acetate uptake rate constant, $K_{is}$ represents the glucose-induced acetate uptake inhibition constant, $K_{sa}$ represents the acetate consumption affinity constant, DOT represents oxygen saturation (%) =$DO_a$/DO*, DOa represents dissolved oxygen concentration (mg/L), DO* represents the saturated dissolved oxygen concentration (mg/L) of the culture solution under the given process conditions, $K_{La}$ represents the oxygen transfer coefficient, qo represents the rate of oxygen consumption, H represents Henry's law constant, $Y_{os}$ represents the glucose/oxygen consumption ratio (yield), $Y_{oa}$ represents acetate/oxygen production yield, $q_{sox}$ represents glucose uptake rate through oxidative metabolism, and $q_m$ represents the cell maintenance constant.

As another example, among the above equations, in Equation 3,

$$p_A = q_{sof} Y_{as}$$

may be replaced with

$$p_A = q_{sof} X Y_{as} X \left(\frac{100 \cdot DOT}{100 + DOT}\right)^i.$$

<Calculation of metabolic model equation parameters>

The data obtained through the culture experiment were substituted into the Escherichia coli metabolic model equation, and a genetic algorithm among known optimization techniques is applied to estimate the parameters. The estimated parameter values are shown in the table below.

[Table 1]

| (Each parameter estimated value) | | | |
|---|---|---|---|
| parameters | value | parameters | value |
| $K_{ap}$ | 0.290 | $q_{Smax}$ | 0.01 |
| $K_{sa}$ | 0.476 | $q_m$ | 0.0396 |
| $K_o$ | 0.973 | $q_{Smax}$ | 1.45 |
| $K_s$ | 1.67e-5 | $Y_{as}$ | 1.03 |
| $K_{ia}$ | 10 | $Y_{xa}$ | 0.1 |
| $K_{is}$ | 0.0366 | $Y_{em}$ | 0.338 |
| $p_{Amax}$ | 0.122 | $Y_{xsof}$ | 0.1 |

<Calculation of predicted substrate concentration>

The predicted substrate concentration was calculated by applying the estimated parameters to Equation 2 above.

(3) Update of parameters

By comparing the actually measured substrate concentration obtained from the culture solution spectrum data with the predicted substrate concentration calculated from the metabolic model equation, the parameters of the metabolic model equation were updated. The parameters of the metabolic model equations in Equations 1 to 4 of the metabolic model unit were updated by applying the genetic algorithm among known optimization algorithms so as to minimize the difference between the actually measured substrate concentration and the substrate concentration according to the substrate metabolism model equation according to Equation 2

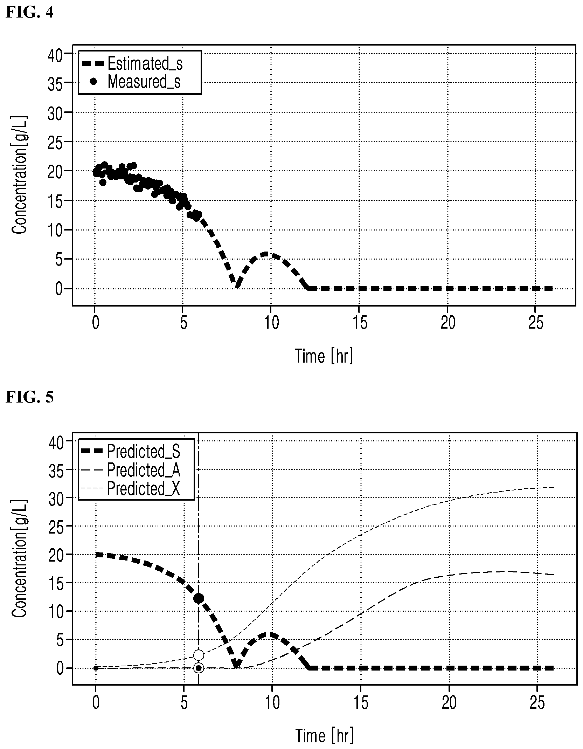

FIG. 4 is a graph illustrating the predicted substrate concentration value Estimated_S using the parameters estimated from the culture experiment data and the actually measured substrate concentration value Meaured_S obtained from the spectrum data. (4) Escherichia coli cell concentration prediction value calculation.

After updating the parameter values, the Escherichia coli cell concentration was predicted from the cell concentration metabolic model equation with updated parameters. FIG. 5 is a graph illustrating the predicted substrate concentration Predicted_S, the predicted acetate concentration Predicted_A, and the predicted Escherichia coli cell concentration Predicted _X values from the metabolic model equation with the updated parameters

**Claims**

1.   A microorganism culture concentration prediction device, comprising:

   a spectral measurement unit that acquires spectrum data of a culture solution of a microorganism incubator; and
   a microorganism production amount prediction unit that calculates an actually measured substrate concentration in a culture solution by inputting the spectrum data into a concentration actual-measurement model and predicts a microorganism culture concentration by updating a parameter of a microorganism metabolism model using the actually measured substrate concentration.

2.   The device of claim 1, wherein
   the microorganism production amount prediction unit is configured to comprise

   a substrate concentration actual-measurement unit that calculates the actually measured substrate concentration from the spectrum data,
   a metabolic model unit that calculates a predicted substrate concentration from a metabolic model equation of a metabolic activity of the microorganism, and
   a parameter update unit that updates the parameters of the metabolic model equation from the actually measured substrate concentration and the predicted substrate concentration.

**3.** The device of claim 2, wherein
the microorganism production amount prediction unit that applies the parameters updated by the parameter update unit to the metabolic model equation, and calculates a microorganism culture concentration predicted value from the metabolic model equation to which the updated parameters are applied.

**4.** The device of claim 2, wherein
the substrate concentration actual-measurement unit comprises a concentration actual-measurement model machine-learned to calculate a concentration of the substrate from the spectrum data, and inputs the real-time spectrum data into the concentration actual-measurement model to calculate the actually measured substrate concentration.

**5.** The device of claim 2, wherein
the metabolic model equation comprises a concentration calculation model equation for predicting a concentration of Escherichia coli cells in the culture solution, and a substrate concentration calculation model equation for calculating a concentration of glucose as a substrate.

**6.** A microorganism culture concentration prediction method in a culture process, the method comprising:

a spectrum data acquisition step of acquiring a real-time spectrum of a culture solution of a microorganism incubator;
an actually measured substrate concentration calculation step of calculating an actually measured substrate concentration in a culture solution by inputting the real-time spectrum data into a pre-trained concentration actual-measurement model;
a predicted substrate concentration calculation step of calculating a predicted substrate concentration of a substrate from a metabolic model equation of the microorganism;
a parameter update step of updating the parameters of the metabolic model equation so that the predicted substrate concentration matches the actually measured substrate concentration by comparing the actually measured substrate concentration with the predicted substrate concentration; and

a microorganism production amount prediction step of predicting a microorganism production amount from the metabolic model equation to which the updated parameters are applied.

**7.** The method of claim 6, wherein
in the predicted substrate concentration calculation step,
the predicted substrate concentration from the metabolic model equation is calculated by estimating the parameters of the metabolic model equation from concentration data of each component in the culture solution obtained in advance, and applying the estimated parameter.

**8.** The method of claim 7, wherein
the metabolic model equation comprises a concentration calculation model equation for predicting the concentration of Escherichia coli cells in the culture solution, and a substrate concentration calculation model equation for calculating a concentration of glucose as a substrate.

**9.** A microorganism culture concentration prediction system, comprising:

an incubator for culturing a microorganism in a culture solution;
a spectrum acquisition unit that acquires a real-time spectrum of the culture solution;
a memory device that stores a concentration actual-measurement model, which is an artificial intelligence model trained to calculate an actually measured substrate concentration from spectrum data, and a metabolic model equation of a metabolic activity of the microorganism; and
a control unit that calculates a predicted value of microorganism production amount from the concentration actual-measurement model and the metabolic model equation, wherein
the control unit

calculates the actually measured substrate concentration by receiving the spectrum data from the spectrum acquisition unit and inputting the spectrum data to the concentration actual-measurement model,
updates parameters of the metabolic model equation from the actually measured substrate concentration and a predicted substrate concentration by calculating the predicted substrate concentration from the metabolic model equation, and

calculates a predicted value of the microorganism production amount from the metabolic model equation with the updated parameters.

**FIG. 1**

**FIG. 2**

(a)

(b)

**FIG. 3**

(a)

(b)

FIG. 4

FIG. 5

## INTERNATIONAL SEARCH REPORT

| International application No. |
|---|
| **PCT/KR2022/012789** |

**A.    CLASSIFICATION OF SUBJECT MATTER**

**C12M 1/34**(2006.01)i; **G01N 21/25**(2006.01)i; **G16C 20/30**(2019.01)i; **G16C 20/70**(2019.01)i

According to International Patent Classification (IPC) or to both national classification and IPC

**B.    FIELDS SEARCHED**

Minimum documentation searched (classification system followed by classification symbols)

C12M 1/34(2006.01); C12M 1/36(2006.01); C12M 3/00(2006.01); C12Q 1/04(2006.01); C12Q 1/06(2006.01); C12Q 1/18(2006.01); G01N 27/62(2006.01); G06Q 50/10(2012.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미생물(microorganism), 배양(culture), 농도(concentration)

**C.    DOCUMENTS CONSIDERED TO BE RELEVANT**

| Category* | Citation of document, with indication, where appropriate, of the relevant passages | Relevant to claim No. |
|---|---|---|
| A | JP 2012-010623 A (HITACHI HIGH-TECHNOLOGIES CORP.) 19 January 2012 (2012-01-19)<br>See claims 1-16. | 1-9 |
| A | JP 2017-504333 A (BRUKER DALTONIK GMBH) 09 February 2017 (2017-02-09)<br>See entire document. | 1-9 |
| A | KR 10-1641588 B1 (CHO, Mun Hwan) 21 July 2016 (2016-07-21)<br>See entire document. | 1-9 |
| A | JP 2007-068547 A (DAIKIN IND LTD.) 22 March 2007 (2007-03-22)<br>See entire document. | 1-9 |
| A | JP 2006-296423 A (HITACHI LTD.) 02 November 2006 (2006-11-02)<br>See entire document. | 1-9 |

☐ Further documents are listed in the continuation of Box C.        ☑ See patent family annex.

| | | |
|---|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family | |

| Date of the actual completion of the international search | Date of mailing of the international search report |
|---|---|
| **06 December 2022** | **07 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.

**PCT/KR2022/012789**

| Patent document cited in search report | | | Publication date (day/month/year) | Patent family member(s) | | | Publication date (day/month/year) |
|---|---|---|---|---|---|---|---|
| JP | 2012-010623 | A | 19 January 2012 | None | | | |
| JP | 2017-504333 | A | 09 February 2017 | CA | 2937285 | A1 | 23 July 2015 |
| | | | | CN | 105916995 | A | 31 August 2016 |
| | | | | CN | 105916995 | B | 25 June 2021 |
| | | | | DE | 102014000646 | A1 | 23 July 2015 |
| | | | | EP | 3094739 | A1 | 23 November 2016 |
| | | | | EP | 3094739 | B1 | 24 October 2018 |
| | | | | ES | 2706517 | T3 | 29 March 2019 |
| | | | | JP | 6769872 | B2 | 14 October 2020 |
| | | | | US | 11142784 | B2 | 12 October 2021 |
| | | | | US | 2016-0333388 | A1 | 17 November 2016 |
| | | | | WO | 2015-107054 | A1 | 23 July 2015 |
| KR | 10-1641588 | B1 | 21 July 2016 | None | | | |
| JP | 2007-068547 | A | 22 March 2007 | CN | 1798849 | A | 05 July 2006 |
| | | | | CN | 1798849 | B | 12 January 2011 |
| | | | | JP | 2005-185278 | A | 14 July 2005 |
| | | | | JP | 2007-125029 | A | 24 May 2007 |
| | | | | JP | 4036236 | B2 | 23 January 2008 |
| | | | | WO | 2005-054498 | A1 | 16 June 2005 |
| JP | 2006-296423 | A | 02 November 2006 | EP | 1705243 | A1 | 27 September 2006 |
| | | | | US | 2006-0216818 | A1 | 28 September 2006 |
| | | | | US | 7771988 | B2 | 10 August 2010 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020207013036

- KR 1020207004943

**Non-patent literature cited in the description**

- **EMMANUEL ANANE.** Modelling overflow metabolism in Escherichia coli by acetate cycling. *Biochemical Engineering Journal,* 2017, vol. 125, 23-30